# FASCICULE DE BREVET EUROPEEN

(11) **EP 1 686 886 B1**
(45) Date de publication et mention de la délivrance du brevet: **17.09.2008**
(21) Numéro de dépôt: 04805479.5
(22) Date de dépôt: 18.11.2004
(51) Int. Cl.: A61B 3/032

(54) **APPAREIL DE DIAGNOSTIC OPHTALMIQUE POUR DIFFERENTS TYPES DE TESTS**
OPHTHALMISCHES DIAGNOSEGERÄT FÜR VERSCHIEDENE ARTEN VON TESTS
OPHTHALMIC DIAGNOSTIC APPARATUS FOR DIFFERENT TYPES OF TESTS

(30) Priorité: 21.11.2003 FR 0313667
(43) Date de publication de la demande: 09.08.2006
(73) Titulaire: ESSILOR INTERNATIONAL (Compagnie Générale d'Optique), F-94220 Charenton-le-Pont (FR)
(72) Inventeur: DIVO, Fabien, F-95160 Montmorency (FR)
(74) Mandataire: Lepelletier-Beaufond, François
(86) Numéro de dépôt international: PCT/FR2004/002942
(87) Numéro de publication internationale: WO 2005/053520

(56) Documents cités:
- US-A- 4 562 433
- US-A- 4 618 231
- US-A- 5 331 358
- US-A- 5 416 540
- US-A- 5 823 958

## Description

L'invention a trait au domaine du matériel ophtalmologique de diagnostic.

Elle concerne plus particulièrement un appareil permettant d'effectuer sur un patient des tests de dépistage d'éventuels défauts de vision tels que l'amétropie, la phorie, ou la dyschromatopsie, et d'effectuer également des mesures telles que la mesure de l'acuité visuelle du patient.

Ce type d'appareil est destiné à afficher différents tests, adaptés aux défauts que l'on souhaite détecter. Le patient est interrogé au fur et à mesure sur ce qu'il voit, et, en fonction de sa réponse, le diagnostic est effectué.

Certains tests sont ainsi colorés (par exemple ceux prévus pour détecter un défaut de vision des couleurs ou de fusion), d'autres représentent des motifs de grande taille (par exemple le "cadran de Parent", pour test d'astigmatisme ou mesure de phorie) et d'autres au contraire des motifs de taille très faible qui de plus doivent être réalisés avec une importante précision (anneaux de Landolt ou autres optotypes pour acuité élevée). Ces tests sont de plus variables suivant les pays.

Il existe donc un très grand nombre de ces tests qui diffèrent les uns des autres par leur couleur, leur taille ou leur précision d'affichage, à tel point qu'il est difficile de réaliser un appareil apte à afficher un grand nombre de tests différents, aux caractéristiques éloignées.

En effet, les test basés sur des optotypes de petite taille, par exemple conformes à la norme ISO 8596, nécessitent une importante précision d'affichage tandis que d'autres tests, par exemple d'astigmatisme, requièrent l'affichage de grandes images ou d'images en couleur, sans nécessiter une précision d'affichage particulière.

La précision d'affichage est obtenue, dans un écran graphique, par une faible taille de pixels, autrement dit par une importante résolution par unité de surface.

Il n'existe pas sur le marché d'écran présentant une résolution permettant d'afficher à la fois des tests nécessitant de la précision et des tests destinés à l'affichage de grand motifs. Cependant, il existe plusieurs possibilités pour afficher différents tests ophtalmologiques. Certains appareils utilisent par exemple une bande sérigraphiée sur laquelle est imprimée l'intégralité des tests. Seule une partie de la bande est affichée dans une fenêtre utile et un système mécanique permet de faire défiler la bande afin d'afficher le test désiré en face de la fenêtre utile.

Ce système est un système mécanique permettant de générer quasiment tous les tests de dépistage et est prévu pour permettre le changement de la bande sérigraphiée pour l'adapter à d'autres pays ou usages.

D'autres appareils similaires mettent en oeuvre des tests imprimés sur un tambour ou un plateau rotatif, la rotation du tambour ou du plateau permettant de présenter le test que l'on souhaite visualiser dans une fenêtre utile.

D'autres appareils encore utilisent des écrans graphiques, par exemple du type CRT (à tube cathodique) ou LCD (à cristaux liquides). Ces écrans permettent de réaliser de très nombreux tests sur une seule surface sans nécessiter de mouvements mécaniques. Il existe ainsi de nombreux logiciels de dépistage, affichant des tests sur des écrans d'ordinateurs conventionnels. Ces tests sont de plus très faciles à modifier ou à adapter suivant les pays, car ils ne nécessitent qu'une modification logicielle. L'inconvénient de ces systèmes est la résolution des écrans, qui est insuffisante pour avoir à la fois une image de taille raisonnable et des pixels assez petits pour réaliser les optotypes de petite taille à la précision requise par exemple par la norme ISO précitée.

Par ailleurs, d'autres appareils sont limités à l'affichage de motifs prédéfinis dans l'écran mais permettent par conséquent un affichage très précis des optotypes de petite taille.

On connaît par ailleurs du brevet américain 4,618,231, qui divulgue les caractéristiques du préambule de la revendication 1, un instrument de mesure de l'amplitude et de la vitesse d'accommodation de l'oeil d'un sujet. Cet instrument comporte un premier écran, fixe, ainsi qu'un second écran, disposé perpendiculairement au premier écran et monté mobile sur un rail. Une lame semi-transparente est rattachée au second écran et est par conséquent mobile avec celui-ci, la lame semi-transparente étant disposée obliquement entre le premier écran et le second écran. Lors de la mesure de l'amplitude d'accommodation, le premier écran n'est pas utilisé et le second écran est constamment en service. Lors de la mesure de la vitesse d'accommodation, le premier écran et le second écran sont utilisés alternativement : les éléments optiques de l'instrument décrit sont configurés de sorte que le premier écran apparaît au sujet en tant qu'objet distant et le second écran lui apparaît en tant qu'objet proche. Dans la mesure de la vitesse d'accommodation dans le sens des objets distants vers les objets proches, le sujet fixe le premier écran illuminé avec le second écran et la lame semi-transparente positionnés à proximité du premier écran. Un générateur d'orientation sélectionne aléatoirement l'une des quatre configurations d'affichage du second écran et éteint le premier écran tandis qu'il allume le second écran. Le sujet observe la configuration du second écran et manipule une palette pour éteindre le second écran et indiquer quelle est l'orientation qui est observée pour le motif affiché par le second écran, ceci étant reproduit pour différentes positions du second écran de plus en plus éloignées du premier écran jusqu'à ce que le sujet commette une erreur d'orientation trois fois de suite. Dans la mesure de la vitesse d'accommodation dans le sens des objets distants vers les objets proches, la procédure est semblable mais le premier écran et le second écran sont inversés.

L'invention a pour but d'améliorer les appareils de diagnostic ophtalmique en permettant l'affichage de tests comportant aussi bien des motifs de grande taille que des optotypes de petite taille à partir d'un affichage graphique configurable.

A cet effet, l'invention vise un appareil de diagnostic ophtalmique selon les revendications 1-14.

D'autres caractéristiques et avantages de l'invention apparaissent à la lumière de la description qui va suivre d'un mode de réalisation préféré donné à titre d'exemple non limitatif, description faite en référence aux dessins annexés dans lesquels :
- La figure 1 est une vue schématique représentant de côté l'oeil d'un patient regardant un appareil de diagnostic selon un premier mode de réalisation de l'invention ;
- la figure 2 représente l'image vue par le patient de la figure 1 ;
- la figure 3 est une vue similaire à la figure 1 représentant un appareil de diagnostic selon un deuxième mode de réalisation de l'invention ;
- la figure 4 est une vue similaire à la figure 2 représentant un appareil de diagnostic selon le deuxième mode de réalisation de l'invention.

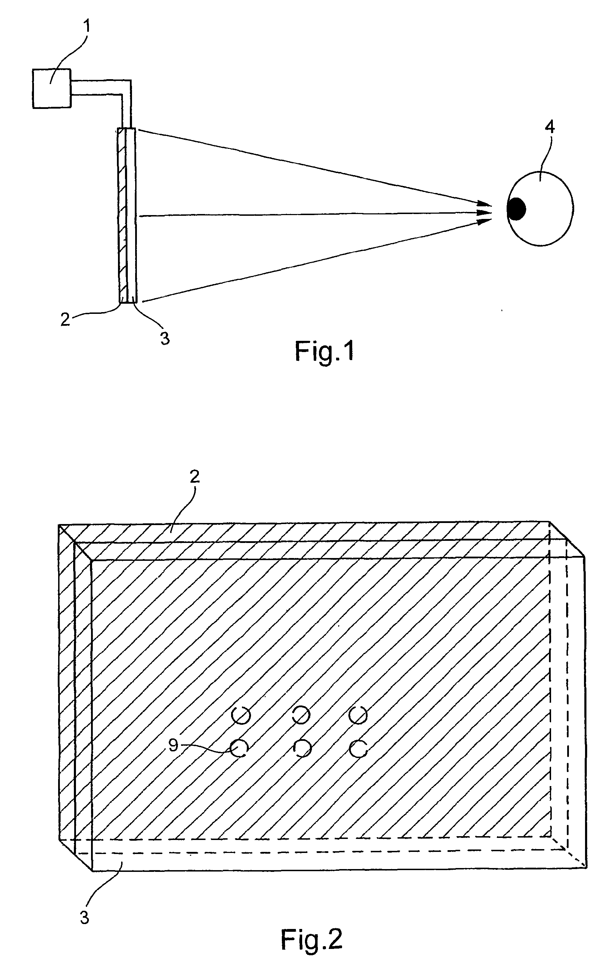

La figure 1 représente schématiquement les principaux éléments d'un appareil de diagnostic ophtalmique. Un module de commande 1 est replié à un premier écran graphique 2 et à un deuxième écran graphique 3. Selon ce premier mode de réalisation, les écrans graphiques 2, 3 sont superposés pour former un afficheur adapté à de nombreux types de tests, y compris en couleur, ainsi qu'à l'affichage de tests nécessitant une précision de réalisation importante, et ce sans mouvement mécanique.

Le premier écran graphique 2 est un écran graphique couleur, du type LCD, CRT ou équivalent, de résolution moyenne, par exemple de 800 lignes par 600 colonnes pour un écran de 15 pouces. Cet écran permet d'afficher des tests nécessitant des couleurs, tels que test de phorie, test de fusion, test duochrome, test d'Hishiara. Il permet aussi d'afficher d'autres types de tests, comme par exemple des tests de défilement, des tests d'acuité (pour les acuités assez basses), ou des tests d'astigmatisme.

Ces différents tests ont en commun de ne pas nécessiter une résolution importante pour être affichés. L'écran graphique 2 peut donc être constitué par un afficheur courant dans le domaine informatique et peu onéreux.

Le deuxième écran graphique 3 permet quant à lui l'affichage de tests nécessitant une grande précision de réalisation, ce que ne peut pas faire le premier écran graphique 2, c'est-à-dire par exemple des tests d'acuité pour les acuités élevées.

Dans cette configuration, le deuxième écran graphique 3 est un écran à cristaux liquides transmissif de même taille que le premier écran graphique 2. Des motifs 9 correspondant à des optotypes pour haute acuité sont gravés directement à la fabrication de l'écran à cristaux liquides. Ces optotypes ne sont donc pas constitués de pixels, ils apparaissent lors de l'activation d'une zone de l'écran à cristaux liquides ayant une forme prédéterminée. Chaque optotype peut être allumé indépendamment des autres.

Lorsque les cristaux liquides du deuxième écran graphique 3 ne sont pas excités, l'écran 3 reste transparent.

Le module de commande 1, constitué par exemple d'un ordinateur et des logiciels adéquats, assure une première fonction d'affichage proprement dit. Il permet d'afficher sur le premier écran 2 certains des tests qui seront choisis par l'utilisateur et permet également d'afficher sur ce premier écran 2 une surface unie, par exemple un fond blanc.

Quant au deuxième écran 3, le module de commande 1 est apte à activer ou désactiver chacune des cellules à cristaux liquides constituant cet écran 3 de manière à faire apparaître certains des optotypes qui y sont gravés ou bien de rendre ce deuxième écran 3 complètement transparent par la désactivation de toutes les cellules.

Le module de commande 1 assure également une deuxième fonction relative à la coordination des affichages du premier écran 2 et du deuxième écran 3. Le module de commande 1 est en effet prévu pour offrir une première configuration d'affichage dans laquelle le premier écran 2 est commandé pour afficher des motifs de tests, le deuxième écran 3 étant maintenu transparent ; et une deuxième configuration d'affichage dans laquelle le premier écran 2 est commandé pour afficher une image blanche unie tandis que le deuxième écran 3 affiche des optotypes gravés.

Dans la première configuration d'affichage, l'oeil 4 d'un patient qui regarde l'appareil de diagnostic ne voit que les tests affichés par le premier écran 2.

Dans la deuxième configuration d'affichage, ce sont les tests affichés par le deuxième écran 3 qui apparaissent par contraste avec le fond que constitue le premier écran 2. Ce dernier est préférentiellement allumé lorsqu'il se trouve comme ici dans son état uni. Ce que voit l'oeil 4 dans cette configuration est représenté à la figure 2 qui montre les deux écrans 2, 3 superposés, les hachures du premier écran 2 montrant que celui-ci est dans sa position « écran blanc » tandis que sur le deuxième écran 3, des optotypes, ici des cercles interrompus 9, apparaissent au premier plan par rapport au fond blanc.

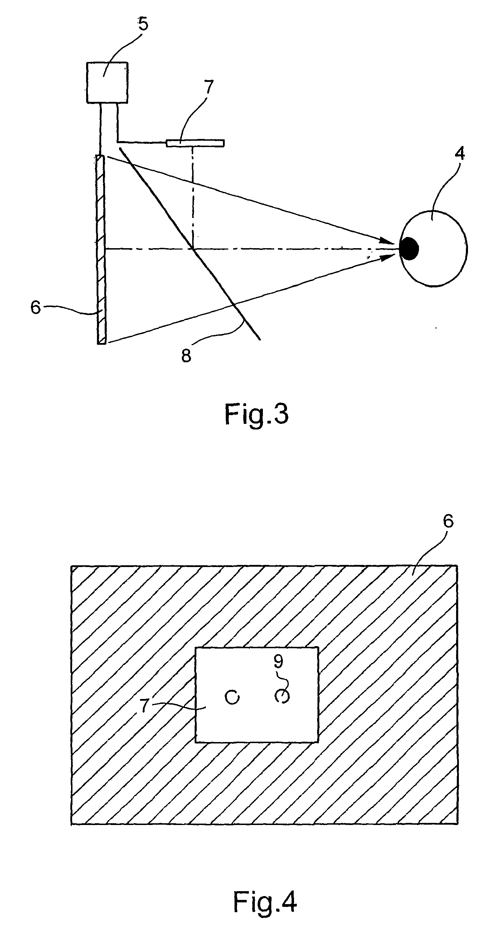

Les figures 3 et 4 correspondent à un deuxième mode de réalisation de l'invention.

L'appareil de diagnostic ophtalmique comporte également un module de commande 5 relié à un premier écran graphique 6 et à un deuxième écran graphique 7. Les écrans graphiques 6, 7 sont disposés perpendiculairement l'un à l'autre et une lame semi-transparente 8 est insérée obliquement entre ces deux écrans 6, 7, en formant un angle d'environ 45° par rapport à chacun des écrans 6, 7.

Le premier écran 6 est similaire au premier écran graphique 2 du premier mode de réalisation des figures 1 et 2. Le deuxième écran graphique 7 est un écran de résolution sensiblement égale à celle du premier écran 6, mais de taille notablement inférieure, le deuxième écran 7 présentant ainsi une résolution par unité de surface supérieure à celle du premier écran 6 (c'est-à-dire que la taille minimale d'un pixel du deuxième écran 7 est inférieure à la taille minimale d'un pixel du premier écran 6).

Le module de commande 5 est ici également adapté à prendre une première configuration dans laquelle le premier écran 6 est activé et affiche des motifs de test tandis que le deuxième écran 7 est éteint. La lame semi-transparente 8, qui a les propriétés d'un miroir sans tain, permet à l'oeil du patient 4 de voir le premier écran 6 mais ne réfléchit pas l'image du deuxième écran 7 qui est éteint.

Dans une deuxième configuration du module de commande 5, l'oeil 4 du patient voit l'image représentée à la figure 4. Le premier écran 6 est alors éteint et le deuxième écran 7 est activé pour afficher des optotypes 9. De même, grâce à la lame semi-transparente 8, l'oeil 4 ne perçoit que les motifs du deuxième écran 7 réfléchis sur la surface de la lame 8. En variante, dans cette deuxième configuration, le premier écran 6 peut également être allumé. Dans ce cas, seule la partie de l'écran 6 sur laquelle ne se superpose pas l'image réfléchie de l'écran 7 doit être allumée.

L'appareil de diagnostic ophtalmique que l'on vient de décrire dans ses premier et deuxième modes de réalisation, est employé de la manière indiquée ci-après.

L'appareil comporte un premier écran graphique 2, 6 dont les caractéristiques (résolution par unité de surface) sont adaptées à l'affichage d'un grand nombre de motifs de tests ophtalmiques qui seront sélectionnés par l'utilisateur grâce à une interface logicielle, et ce à moindre coût.

Le deuxième écran graphique 3, 7 est adapté à l'affichage des tests nécessitant une grande précision de réalisation.

Ces deux écrans 2, 3, 6, 7 sont superposés, physiquement ou optiquement et sont coordonnés grâce à un module de commande 1, 5 qui permet de rendre l'un des écrans actif tandis que l'autre reste inactif. L'utilisateur peut directement sélectionner quelle configuration du module de commande 1, 5 est la plus adaptée. Le module de commande 1, 5 peut de plus sélectionner automatiquement la configuration adéquate correspondant au test sélectionné par l'utilisateur.

## Revendications

1. Appareil de diagnostic ophtalmique comportant un premier organe à regarder (2, 6) depuis un emplacement prédéterminé (4) et un deuxième organe à regarder (3, 7, 8) depuis ledit emplacement (4), adaptés chacun à afficher des motifs de tests ophtalmiques, le deuxième organe à regarder (3, 7, 8) étant disposé entre le premier organe à regarder (2, 6) et l'emplacement prédéterminé (4), lequel premier organe à regarder (2, 6) admet un état uni et un état matérialisant des signes et lequel deuxième organe à regarder (3, 7, 8) admet un état transparent et un état matérialisant des signes ; et comportant en outre un module de commande (1, 5) des premier et deuxième organes à regarder adapté à leur faire prendre une première configuration dans laquelle le deuxième organe à regarder (3, 7, 8) est dans son état matérialisant des signes et le premier organe à regarder (2, 6) est dans son état uni, et une deuxième configuration dans laquelle le deuxième organe à regarder (3, 7, 8) est dans son état transparent et le premier organe à regarder (2, 6) est dans son état matérialisant des signes ; **caractérisé en ce que** l'un des organes à regarder est adapté à l'affichage de différents tests qui ne nécessitent pas une résolution importante pour être affichés et l'autre des organes à regarder est un écran à cristaux liquides comprenant des optotypes pour haute acuité qui y sont gravés directement, ne sont pas constitués de pixels, et sont allumables indépendamment les uns des autres.

2. Appareil de diagnostic ophtalmique comportant un premier organe à regarder (2, 6) depuis un emplacement prédéterminé (4) et un deuxième organe à regarder (3, 7, 8) depuis ledit emplacement (4), adaptés chacun à afficher des motifs de tests ophtalmiques, le deuxième organe à regarder (3, 7, 8) étant disposé entre le premier organe à regarder (2, 6) et l'emplacement prédéterminé (4), lequel premier organe à regarder (2, 6) admet un état uni et un état matérialisant des signes et lequel deuxième organe à regarder (3, 7, 8) admet un état transparent et un état matérialisant des signes ; et comportant en outre un module de commande (1, 5) des premier et deuxième organes à regarder adapté à leur faire prendre une première configuration dans laquelle le deuxième organe à regarder (3, 7, 8) est dans son état matérialisant des signes et le premier organe à regarder (2, 6) est dans son état uni, et une deuxième configuration dans laquelle le deuxième organe à regarder (3, 7, 8) est dans son état transparent et le premier organe à regarder (2, 6) est dans son état matérialisant des signes ; **caractérisé en ce que** l'un des organes à regarder est adapté à l'affichage de différents tests qui ne nécessitent pas une résolution importante pour être affichés et l'autre des organes à regarder a une taille minimale d'un pixel qui est notablement inférieure à une taille minimale d'un pixel dudit un des organes à regarder.

3. Appareil selon l'une des revendications 1 et 2, **caractérisé en ce que** l'organe à regarder présentant la résolution plus importante est disposé entre l'autre organe à regarder et ledit emplacement.

4. Appareil selon la revendication 1 ou les revendications 1 et 3, **caractérisé en ce que** ledit premier organe à regarder (2, 6) et ledit deuxième organe à regarder (3, 7, 8) sont superposés physiquement, et ce sans mouvement mécanique.

5. Appareil selon la revendications 2 ou les revendications 2 et 3, **caractérisé en ce que** ledit premier organe à regarder (2, 6) et ledit deuxième organe à regarder (3, 7, 8) sont superposés optiquement pour former un afficheur adapté à une pluralité de tests ophtalmiques.

6. Appareil selon l'une des revendications 1 à 5, **caractérisé en ce que** le premier organe à regarder comporte un premier écran graphique (2, 6).

7. Appareil selon l'une des revendications 1 à 6, **caractérisé en ce que** le deuxième organe à regarder comporte un deuxième écran graphique (3, 7).

8. Appareil selon les revendications 1, 6 et 7, **caractérisé en ce que** le premier écran graphique (2) et le deuxième écran graphique (3) sont sensiblement parallèles.

9. Appareil selon la revendication 8, **caractérisé en ce que** le premier écran graphique (2) et le deuxième écran graphique (3) sont superposés.

10. Appareil selon l'une des revendications 8 et 9, **caractérisé en ce que** le premier écran graphique est un écran couleur (2) et **en ce que** le deuxième écran graphique est un afficheur à cristaux liquides (3).

11. Appareil selon la revendication 10, **caractérisé en ce que** le deuxième écran graphique comporte un afficheur à cristaux liquides (3) gravé de motifs de tests ophtalmiques prédéfinis (9).

12. Appareil selon les revendications 2, 6 et 7, **caractérisé en ce que** le deuxième organe à regarder comporte, en outre du deuxième écran graphique (7), un organe réfléchissant (8) adapté à refléter le deuxième écran graphique (7).

13. Appareil selon la revendication 12, **caractérisé en ce que** le premier écran graphique (6), appartenant au premier organe à regarder, est disposé perpendiculairement au deuxième écran graphique (7) appartenant au deuxième organe à regarder, et **en ce que** l'organe réfléchissant comporte une lame semi-transparente (8) disposée obliquement par rapport aux deux écrans graphiques (6, 7).

14. Appareil selon la revendication 13, **caractérisé en ce que** le deuxième écran graphique (7) présente une surface inférieure à celle du premier écran graphique (6).

## Claims

1. Ophthalmic diagnostic apparatus comprising a first body (2, 6) to be viewed from a predetermined position (4) and a second body (3, 7, 8) to be viewed from said position (4), each body being adapted to display ophthalmic test patterns, the second body (3, 7, 8) to be viewed being disposed between the first body (2, 6) to be viewed and the predetermined position (4), which first body (2, 6) to be viewed has a uniform state and a state for showing signs and which second body (3, 7, 8) to be viewed has a transparent state and a state for showing signs, and further comprising a control module (1, 5) for the first and second bodies to be viewed adapted to cause them to assume a first configuration in which the second body (3, 7, 8) to be viewed is in its state for showing signs and the first body (2, 6) to be viewed is in its uniform state and a second configuration in which the second body (3, 7, 8) to be viewed is in its transparent state and the first body (2, 6) to be viewed is in its state for showing signs,
**characterised in that** one of the bodies to be viewed is suitable for displaying various tests that do not require a high resolution to be displayed and the other of the bodies to be viewed is a liquid crystal screen comprising optotypes for high acuity that are etched directly therein, do not consist of pixels and can be turned on independently of one another.

2. Ophthalmic diagnostic apparatus comprising a first body (2, 6) to be viewed from a predetermined position (4) and a second body (3, 7, 8) to be viewed from said position (4), each body being adapted to display ophthalmic test patterns, the second body (3, 7, 8) to be viewed being disposed between the first body (2, 6) to be viewed and the predetermined position (4), which first body (2, 6) to be viewed has a uniform state and a state for showing signs and which second body (3, 7, 8) to be viewed has a transparent state and a state for showing signs, and further comprising a control module (1, 5) for the first and second bodies to be viewed adapted to cause them to assume a first configuration in which the second body (3, 7, 8) to be viewed is in its state for showing signs and the first body (2, 6) to be viewed is in its uniform state and a second configuration in which the second body (3, 7, 8) to be viewed is in its transparent state and the first body (2, 6) to be viewed is in its state for showing signs,
**characterised in that** one of the bodies to be viewed is suitable for displaying various tests that do not require a high resolution to be displayed and the other of the bodies to be viewed has a minimum size of a pixel that is appreciably smaller than a minimum size of a pixel of said one of the bodies to be viewed.

3. Apparatus according to either of claims 1 and 2, **characterised in that** the body to be viewed having the higher resolution is placed between the other body to be viewed and said position.

4. Apparatus according to claim 1 or claims 1 and 3, **characterised in that** said first body (2, 6) to be viewed and said second body (3, 7, 8) to be viewed are physically superposed, this being accomplished without any mechanical movement.

5. Apparatus according to claim 2 or claims 2 and 3, **characterised in that** said first body (2, 6) to be viewed and said second body (3, 7, 8) to be viewed are optically superposed so as to form a display suitable for a plurality of ophthalmic tests.

6. Apparatus according to one of claims 1 to 5, **characterised in that** the first body to be viewed comprises a first graphics screen (2, 6).

7. Apparatus according to one of claims 1 to 6, **characterised in that** the second body to be viewed comprises a second graphics screen (3, 7).

8. Apparatus according to claims 1, 6 and 7, **characterised in that** the first graphics screen (2) and the second graphics screen (3) are substantially parallel.

9. Apparatus according to claim 8, **characterised in that** the first graphics screen (2) and the second graphics screen (3) are superposed.

10. Apparatus according to either claim 8 or claim 9, **characterised in that** the first graphics screen is a colour screen (2) and the second graphics screen is a liquid crystal display (3).

11. Apparatus according to claim 10, **characterised in that** the second graphics screen comprises a liquid crystal display (3) etched with predefined ophthalmic test patterns (9).

12. Apparatus according to claims 2, 6 and 7, **characterised in that** the second body to be viewed comprises a second graphics screen (7) and a reflecting body (8) adapted to reflect the second graphics screen (7).

13. Apparatus according to claim 12, **characterised in that** a first graphics screen (6) belonging to the first body to be viewed is perpendicular to the second graphics screen (7) belonging to the second body to be viewed and the reflecting body comprises a semitransparent sheet (8) disposed obliquely to the two graphics screens (6, 7).

14. Apparatus according to claim 13, **characterised in that** the second graphics screen (7) has a smaller area than the first graphics screen (6).

## Patentansprüche

1. Ophthalmisches Diagnosegerät, umfassend ein von einem vorbestimmten Ort (4) zu beobachtendes erstes Objekt (2, 6), und ein von dem Ort (4) zu beobachtendes zweites Objekt (3, 7, 8), jeweils ausgelegt um ophthalmische Testmotive anzuzeigen, wobei das zweite zu beobachtende Objekt (3, 7, 8) angeordnet ist zwischen dem ersten zu beobachtenden Objekt (2, 6) und dem vorbestimmten Ort (4), wobei das erste zu beobachtende Objekt (2, 6) einen Uni-Zustand und einen Zustand der Zeichen wiedergibt ermöglicht, und wobei das zweite zu beobachtende Objekt (3, 7, 8) einen transparenten Zustand und einen Zustand der Zeichen wiedergibt ermöglicht; und umfassend ferner ein Steuermodul (1, 5) der ersten und zweiten zu beobachtenden Objekte, ausgelegt um sie zu veranlassen, eine erste Konfiguration einzunehmen, in welcher das zweite zu beobachtende Objekt (3, 7, 8) in seinem Zustand vorliegt, in welchem Zeichen wiedergegeben werden und in welcher das erste zu beobachtende Objekt (2, 6) in seinem einfarbigen oder Uni-Zustand vorliegt, sowie eine zweite Konfiguration, in welcher das zweite zu beobachtende Objekt (3, 7, 8) in seinem transparenten Zustand vorliegt und erste zu beobachtende Objekt (2, 6) in seinem Zustand vorliegt, in welchem Zeichen wiedergegeben werden, **dadurch gekennzeichnet, dass** eines der zu beobachtenden Objekte geeignet ist zum Anzeigen von unterschiedlichen Tests, die keine beachtliche Auflösung erfordern, um angezeigt zu werden, wobei das andere der zu beobachtenden Objekte ein Flüssigkristallbildschirm ist, umfassend Optotypen für hohe Schärfe, die unmittelbar graviert vorliegen, nicht mittels Pixeln gebildet, und unabhängig voneinander einschaltbar sind.

2. Ophthalmisches Diagnosegerät, umfassend ein von einem vorbestimmten Ort (4) zu beobachtendes erstes Objekt (2, 6), und ein von dem Ort (4) zu beobachtendes zweites Objekt (3, 7, 8), jeweils ausgelegt um ophthalmische Testmotive anzuzeigen, wobei das zweite zu beobachtende Objekt (3, 7, 8) angeordnet ist zwischen dem ersten zu beobachtenden Objekt (2, 6) und dem vorbestimmten Ort (4), wobei das erste zu beobachtende Objekt (2, 6) einen Uni-Zustand und einen Zustand der Zeichen wiedergibt ermöglicht, und wobei das zweite zu beobachtende Objekt (3, 7, 8) einen transparenten Zustand und einen Zustand der Zeichen wiedergibt ermöglicht; und umfassend ferner ein Steuermodul (1, 5) der ersten und zweiten zu beobachtenden Objekte, ausgelegt um sie zu veranlassen, eine erste Konfiguration einzunehmen, in welcher das zweite zu beobachtende Objekt (3, 7, 8) in seinem Zustand vorliegt, in welchem Zeichen wiedergegeben werden und in welcher das erste zu beobachtende Objekt (2, 6) in seinem einfarbigen oder Uni-Zustand vorliegt, sowie eine zweite Konfiguration, in welcher das zweite zu beobachtende Objekt (3, 7, 8) in seinem transparenten Zustand vorliegt und erste zu beobachtende Objekt (2, 6) in seinem Zustand vorliegt, in welchem Zeichen wiedergegeben werden, **dadurch gekennzeichnet, dass** eines der zu beobachtenden Objekte ausgelegt ist zum Anzeigen unterschiedlicher Tests, die keine beachtliche Auflösung benötigen, um angezeigt zu werden, wobei das andere der zu beobachtenden Objekte eine minimale Größe eines Pixels aufweist, die insbesondere nennenswert kleiner als eine minimale Größe eines Pixels des genannten einen zu beobachtenden Objektes ist.

3. Gerät nach einem der Ansprüche 1 und 2, **dadurch gekennzeichnet, dass** das zu beobachtende Objekt, welches einen größere Auflösung aufweist, angeordnet ist, zwischen dem zu beobachtenden Objekt und dem Ort.

4. Gerät nach Anspruch 1 oder den Ansprüchen 1 und 3, **dadurch gekennzeichnet, dass** das erste zu beobachtende Objekt (2, 6) und das zweite zu beobachtende Objekt (3, 7, 8) physikalisch überlagert sind und dies ohne mechanische Bewegung.

5. Gerät nach Anspruch 2 oder den Ansprüchen 2 und 3, **dadurch gekennzeichnet, dass** das erste zu beobachtende Objekt (2, 6) und das zweite zu beobachtende Objekt (3, 7, 8) optisch überlagert sind, um eine Anzeige zu bilden, geeignet für eine Vielzahl von ophthalmischen Tests.

6. Gerät nach einem der Ansprüche 1 bis 5, **dadurch gekennzeichnet, dass** das erste zu beobachtende Objekt einen ersten graphischen Schirm (2, 6) umfasst.

7. Gerät nach einem der Ansprüche 1 bis 6, **dadurch gekennzeichnet, dass** das zweite zu beobachtende Objekt einen zweiten graphischen Schirm (3, 7) umfasst.

8. Gerät nach den Ansprüchen 1, 6 und 7, **dadurch gekennzeichnet, dass** der erste graphische Schirm (2) und der zweite graphische Schirm (3) im Wesentlichen parallel vorliegen.

9. Gerät nach Anspruch 8, **dadurch gekennzeichnet, dass** der erste graphische Schirm (2) und der zweite graphische Schirm (3) überlagert vorliegen.

10. Gerät nach einem der Ansprüche 8 und 9, **dadurch gekennzeichnet, dass** der erste graphische Schirm ein Farbschirm (2) ist, und **dadurch**, dass der zweite graphische Schirm eine Flüssigkristallanzeige (3) ist.

11. Gerät nach Anspruch 10, **dadurch gekennzeichnet, dass** der zweite graphische Schirm eine Flüssigkristallanzeige (3) umfasst, graviert mit vorbestimmten ophthalmischen Testmotiven (9).

12. Gerät nach den Ansprüchen 2, 6 und 7, **dadurch gekennzeichnet, dass** das zweite zu beobachtende Objekt neben dem zweiten graphischen Schirm (7) ein Beugungselement (8) umfasst, ausgelegt zum Spiegeln des zweiten graphischen Schirmes (7).

13. Gerät nach Anspruch 12, **dadurch gekennzeichnet, dass** der erste graphische Schirm (6) zugehörig zum ersten zu beobachtenden Objekt senkrecht angeordnet ist bezüglich des zweiten graphischen Schirmes (7), zugehörig zu dem zweiten zu beobachtenden Objekt, und **dadurch**, dass das Beugeelement ein semitransparentes Blatt (8) umfasst, schräg angeordnet, mit Bezug auf die zwei graphischen Schirme (6, 7).

14. Gerät nach Anspruch 13, **dadurch gekennzeichnet, dass** der zweite graphische Schirm (7) eine kleinere Fläche aufweist, als der erste graphische Schirm (6).
